# EUROPEAN PATENT APPLICATION

(11) **EP 3 449 871 A2**
(43) Date of publication of application: **06.03.2019**
(21) Application number: 18275132.1
(22) Date of filing: 24.08.2018
(51) Int. Cl.: A61F 2/07, A61F 2/24, A61F 2/89

(54) **ENDOLUMINAL PROSTHESIS WITH AN AORTIC SINUS STENT ASSEMBLY**

(30) Priority: 01.09.2017 US 201762553235 P
(71) Applicant: Cook Medical Technologies LLC, Bloomington, IN 47404 (US)
(72) Inventor: LUKAS, Saylan, Cincinnati, OH 45244 (US); ELLER, Derek, Orient, OH 43146 (US); KRATZBERG, Jarin A., Lafayette, IN 47909 (US); ROEDER, Blayne A., Lafayette, IN 47909 (US)
(74) Representative: Williams Powell

(57) **Abstract**

A prosthesis for placement within an aortic root, includes a graft having a proximal end, distal end, and lumen disposed therethrough and a stent assembly (50) disposed at that proximal end of the graft, the stent assembly (50) comprising a first stent unit (52a) comprising a first proximal section (54) having a first bend (58) interconnected by a first curved strut and a second curved strut and a second bend (60) interconnected by a third curved strut and a fourth curved strut, the first bend (58) of the first proximal section (54) and second bend (60) of the first proximal section (54) facing each other and circumferentially spaced apart; and a first distal section connected to the first proximal section.

## Description

### BACKGROUND

The functional vessels of human and animal bodies, such as blood vessels and ducts, occasionally weaken or even rupture. For example, the aortic wall can weaken, resulting in an aneurysm, or it may develop a tear in one of the layers of the aortic wall resulting in an aortic dissection.

One common surgical intervention for weakened, aneurysmal or ruptured passageways or ducts involves the use of an endoluminal prosthesis to provide some or all of the functionality of the original, healthy passageway or duct and/or preserve any remaining vascular integrity by replacing a length of the existing passageway or duct wall that spans the site of failure or defect. Endoluminal prostheses may be of a unitary construction or may be comprised of multiple prosthetic modules. They also may be a single tubular device or a bifurcated branching device depending on the desired application.

In many cases, however, the damaged or defected portion of the vasculature may include a branch vessel branching from the main vessel. For example, in the case of the abdominal aorta, there are at least three major branch vessels, including the celiac, mesenteric, and renal arteries, as well as other others, leading to various other body organs. Thus, when the damaged portion of the vessel includes one or more of these branch vessels, some accommodation must be made to ensure that the prosthesis does not block or hinder blood flow through the branch vessel. In many instances, there may in insufficient healthy tissue in the aorta near the branching vessels adequately seal a prosthesis without partially or completely blocking one or more of the branching vessels.

The thoracic aorta presents a challenging anatomy for stent grafts used to treat thoracic aneurysms or dissections. The thoracic aorta comprises a curve known as the aortic arch, which extends between the ascending thoracic aorta (closet to the heart) and the descending thoracic aorta (which extends toward the abdominal aorta). Thoracic stent grafts are used to exclude thoracic aortic aneurysms. A stent graft's ability to conform to the tortuous anatomy of the aortic arch is a major concern. Current designs sometimes lack the desired sealing ability at the proximal end of the stent graft (closest to the heart). Also, current thoracic devices present a relatively large profile which, with some patients' anatomies may be problematic. Finally, many current stents have relatively acute points that may prevent them from being used in the aortic arch for fear of undesirable interaction with the artery wall after an extended amount of time in the patient.

As one particular example, type-A thoracic aortic dissection (TAD-A) is a condition in which the intimal layer of the ascending thoracic aorta develops a tear, allowing blood to flow into the layers of the aortic wall, causing the development of a medial or subintimal hematoma. TAD-A is associated with a strikingly high mortality rate (about one-fourth to one-half of victims die within the first 24-48 hours). The current treatment for TAD-A is open surgery, where the chest is opened, the aorta is clamped, and a vascular prosthesis is sewn in place. Operative mortality rate for this procedure may be around 10%. Endovascular treatment of TAD-B (which affects the descending thoracic aorta) has been effective in reducing short-term and longer term mortality. Treatment of TAD-A may offer benefits as well, but is challenged by the likelihood that a graft or stent graft in the ascending aorta may migrate proximally toward the heart or distally away from it due to the turbulence of blood flow and the motion associated with the heart beating, thereby blocking coronary or great arteries, respectively.

### BRIEF SUMMARY

Aspects of the present invention seek to provide an improved prosthesis.

According to an aspect of the invention, there is provided a prosthesis as in claim 1.

According to an aspect of the invention, there is provided a stent as in claim 10.

According to an aspect of the invention, there is provided a prosthesis as in claim 15.

Embodiments can provide an endovascular device configured to address the anatomic challenges of the ascending thoracic aorta including preventing migration of the device.

In one aspect, a prosthesis for placement within an aortic root, includes a graft having a proximal end, distal end, and lumen disposed therethrough and a stent assembly disposed at that proximal end of the graft, the stent assembly comprising a first stent unit comprising a first proximal section having a first bend interconnected by a first curved strut and a second curved strut and a second bend interconnected by a third curved strut and a fourth curved strut, the first bend of the first proximal section and second bend of the first proximal section facing each other and circumferentially spaced apart; and a first distal section connected to the first proximal section. In some embodiments, the first distal section comprises a plurality of structural struts connected by apices. In other embodiments, the stent further includes a second proximal section circumferentially adjacent to the first proximal section, the second proximal section comprising a first bend interconnected by a first curved strut and a second curved strut and second bend interconnected by a third curved strut and a fourth curved strut, the first bend of the second proximal section and the second bend of the second proximal section facing each other and circumferentially disposed. In other embodiments, the stent assembly includes a third proximal section circumferentially adjacent to the first proximal section and the second proximal section, the second section comprising a first bend and second bend, the first bend and the second bend facing each other and circumferentially disposed. In other embodiments, at least one fenestration is disposed through a side wall of the graft. The at least one fenestration may be positioned between the first proximal section and the second proximal section of the stent assembly and may be pivotable in any direction away from an axis perpendicular to a longitudinal axis of the prosthesis.

In another aspect, a stent for an aortic valve sinus, includes a stent assembly disposed at that proximal end of the graft, the stent assembly comprising a first stent unit comprising a first proximal section having a first bend interconnected by a first curved strut and a second curved strut and a second bend interconnected by a third curved strut and a fourth curved strut, the first bend of the first proximal section and second bend of the second proximal section facing each other and circumferentially spaced apart; and a distal section comprising a plurality of structural struts connected by apices. In other embodiments, the stent further includes a second proximal section circumferentially adjacent to the first proximal section, the second proximal section having a first bend interconnected by a first curved strut and a second curved strut and second bend interconnected by a third curved strut and a fourth curved strut, the first bend of the second proximal section and the second bend of the second proximal section facing each other and circumferentially disposed. In other embodiments, the stent assembly includes a third stent unit including a third proximal section circumferentially adjacent to the first proximal section and the second proximal section, the second section having a first bend and second bend, the first bend and the second bend facing each other and circumferentially disposed.
In yet another aspect, a prosthesis for placement within an aortic sinus includes a graft having a proximal end, a distal end, and a lumen disposed therethrough, a valve replacement positioned between the proximal end of the graft and the distal end of the graft, and a stent assembly disposed at the proximal end of the graft, the stent assembly comprising at least one proximal section, the proximal section comprising a first proximal apex interconnected by struts and a second proximal apex interconnected by struts.

### BRIEF DESCRIPTION OF THE DRAWINGS

Embodiments of the invention are described below, by way of example only, with reference to the accompanying drawings.
Figure 1 shows an embodiment of an endoluminal prosthesis having pivotable fenestrations comprising an aortic sinus stent assembly.
Figure 2 is an alternative view of the prosthesis of Figure 1.
Figure 3 shows a perspective view of an embodiment of an aortic sinus stent assembly.
Figure 4 is a top view of the aortic sinus stent assembly of Figure 3.
Figures 5 and 6 are side views of an exemplary delivery system that may be used to deliver the endoluminal prosthesis of FIG. 1.
Figure 7A and 7B shows an embodiment of an endoluminal prosthesis having pivotable fenestrations comprising an aortic valve stent assembly deployed in an aortic root of a patient.
Figure 8 shows another embodiment of an endoluminal prosthesis having pivotable fenestrations comprising an aortic sinus stent assembly.

### DETAILED DESCRIPTION OF THE DRAWINGS AND THE PRESENTLY PREFERRED EMBODIMENTS

The present disclosure relates to an endoluminal prosthesis, such as a stent graft that includes one or more fenestrations to accommodate endovascular disease, such as an aneurysm in cases where one or more side branches is involved, and a side branch prosthesis is deployed within the fenestration to permit fluid flow from the endoluminal prosthesis into the branch vessel. The prosthesis includes fenestrations that pivot as needed to accommodate the dynamic geometry of the aortic branches. In various embodiments shown and described in more detail below, for example, one or more pivotable fenestrations provided on a prosthesis lie outside the surface plane of the body of the prosthesis and will allow a branch vessel stent, graft or stent-graft that has been placed in the fenestration to pivot into a variety of orientations required to meet and seal the branch vessel device in the branch vessel. The orientation of the fenestrations may dynamically change over time as needed by changing anatomy.

Unless defined otherwise, all technical and scientific terms used herein have the same meaning as commonly understood to one of ordinary skill in the art to which this invention belongs.

The term "distal" means a location or direction that is, or a portion of a device that when implanted is further downstream in the direction of or with respect to blood flow.

The term "proximal" means a location or direction that is, or a portion of a device that when implanted is further upstream in the direction of or with respect to blood flow.

The term "fenestration" means an opening provided through a surface of a prosthesis from the interior of the prosthesis to the exterior of the prostheses and may have a variety of geometries, including circular, semi-circular, oval, oblong, as well as other geometries.

The term "biocompatible" refers to a material that is substantially non-toxic in the in vivo environment of its intended use, and that is not substantially rejected by the patient's physiological system (i.e., is non-antigenic). Examples of biocompatible materials from which textile graft material can be formed include, without limitation, polyesters, such as polyethylene terephthalate; fluorinated polymers, such as polytetrafluoroethylene (PTFE) and fibers of expanded PTFE, and polyurethanes. In addition, materials that are not inherently biocompatible may be subjected to surface modifications in order to render the materials biocompatible. Examples of surface modifications include graft polymerization of biocompatible polymers on the materials surface, coating of the surface with a crosslinked biocompatible polymer, chemical modification with biocompatible functional groups, and immobilization of a compatibilizing agent such as heparin or other biocompatible substances. Thus, any fibrous material having sufficient strength to survive in the in vivo environment may be used to form a textile graft, provided the final textile is biocompatible. Fibers suitable for making textile grafts include polyethylene, polypropylene, polyaramids, polyacrylonitrile, nylon, and cellulose, in addition to the polyesters, fluorinated polymers, and polyurethanes as listed above. Furthermore, bioremodelable materials may also be used singly or in combination with the aforementioned polymer materials. The textile may be made of one or more polymers that do not require treatment or modification to be biocompatible. The graft may be constructed from woven multifilament polyester, for example and without limitation, Dacron™, produced by DuPONT. Dacron™ is known to be sufficiently biologically inert, non-biodegradable, and durable to permit safe insertion inside the human body.

The term "prosthesis" means any device for insertion or implantation into or replacement for a body part or function of that body part. It may also mean a device that enhances or adds functionality to a physiological system. The term prosthesis may include, for example and without limitation, a stent, stent-graft, filter, valve, balloon, embolization coil, and the like.

The term "tubular" refers to the general shape of an endoluminal device which allows the module to carry fluid along a distance or fit within a tubular structure such as an artery. Tubular prosthetic devices include single, branched, and bifurcated devices. Tubular may refer to any shape including, but not limited to, tapered, cylindrical, curvilinear, or any combination thereof. A tubular device may have a cross-sectional shape that is, circular, substantially circular or the like. However, it should be understood that the cross-sectional shape is not limited thereto, and other shapes, such as, for example, hexagonal, pentagonal, octagonal, or the like are contemplated. The term "endoluminal" refers to or describes objects that can be placed inside a lumen or a body passageway in a human or animal body. A lumen or a body passageway can be an existing lumen or a lumen created by surgical intervention. As used in this specification, the terms "lumen" or "body passageway" are intended to have a broad meaning and encompasses any duct (e.g., natural or iatrogenic) within the human body and can include a member selected from the group comprising: blood vessels, respiratory ducts, gastrointestinal ducts, and the like. "Endoluminal device" or "endoluminal prosthesis" thus describes devices that can be placed inside one of these lumens.

The term "graft" or "graft material" describes an object, device, or structure that is joined to or that is capable of being joined to or implanted in or against a body part to enhance, repair, or replace a portion or a function of that body part. A graft by itself or with the addition of other elements, such as structural components, may comprise an endoluminal prosthesis. The graft may be comprised of a single material, a blend of materials, a weave, a laminate, or a composite of two or more materials. The graft may be constructed from natural or organic materials, for example and without limitation, a biological scaffold or bioremodelable material, such as small intestine submucosa ("SIS"), which is commercially available by Cook Biotech, West Lafayette, IN. The graft may also be constructed from a synthetic, for example and without limitation, a polymer. The graft may be formed from a single layer or multiple layers of material. In embodiments employing a plurality of layers of material, the layers may remain separate, or may be attached to each other through a secondary process such as sintering, curing, adhesives, and sutures or the like.

The term "stent" means any device or structure that adds rigidity, expansion force or support to a prosthesis. A stent is used to obtain and maintain the patency of the body passageway while maintaining the integrity of the passageway. Also, the stent may be used to form a seal. The stent may be located on the exterior of the device, the interior of the device, or both. A stent may be self-expanding, balloon-expandable or may have characteristics of both. A variety of other stent configurations are also contemplated by the use of the term "stent." The stents 16 may be comprised of a metallic material selected from stainless steel, silver, platinum, palladium, gold, titanium, tantalum, iridium, tungsten, cobalt, chromium, cobalt-chromium alloy 1058, cobalt-based 35N alloy, nickel-based alloy 625, a molybdenum alloy, a molybdenum alloy including about 0.4% to about 0.8% of lanthanum oxide (Li₂O₃), and a nickel-titanium alloy, such as Nitinol, or other suitable materials as known in the art. The stents may be made of a wire, or may be laser or cannula cut, or manufactured by other known methods.

The term "branch vessel" refers to a vessel that branches off from a main vessel. Examples are the celiac and renal arteries which are branch vessels to the aorta (i.e., the main vessel in this context). As another example, the hypogastric artery is a branch vessel to the common iliac, which is a main vessel in this context. Thus, it should be seen that "branch vessel" and "main vessel" are relative terms.

"Longitudinally" refers to a direction, position or length substantially parallel with a longitudinal axis of a reference, and is the length-wise component of the helical orientation.

"Circumferentially" refers to a direction, position, or length that encircles a longitudinal axis of reference. The term "circumferential" is not restricted to a full 360° circumferential turn or to a constant radius.

The terms "patient," "subject," and "recipient" as used in this application refer to any animal, especially humans.

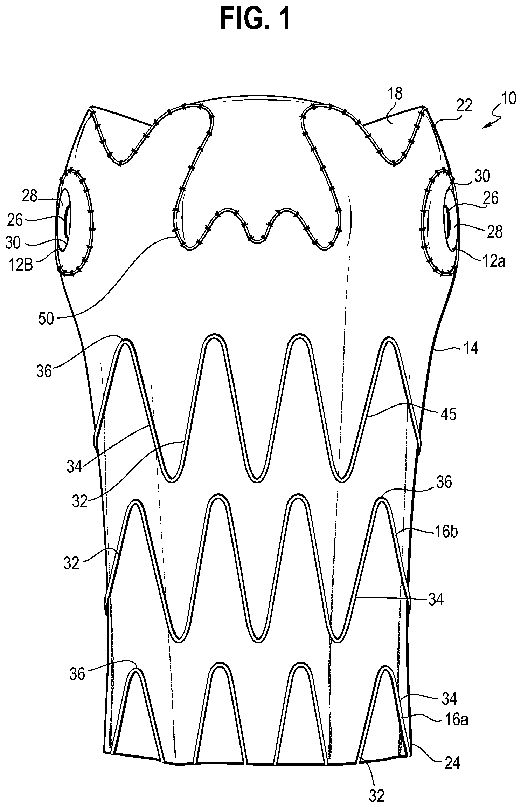

The Figures 1-8 show various embodiments of a prosthesis having pivotable fenestrations and an aortic root sealing stent. The fenestrated prosthesis 10 has a generally tubular body and comprising a biocompatible material, having one or more fenestrations 12 pivotable in a direction away from an axis perpendicular to a longitudinal axis of the prosthesis. For example, the fenestrations 12 may be pivotable in any direction away from an axis perpendicular to a longitudinal axis of the prosthesis 10. The pivotable fenestrations 12 include a first, inner perimeter 26 surrounding the fenestration 12 having a diameter, a band 28 of flexible material attached to and surrounding the first perimeter 26, and a second, outer perimeter 30 attached to and surrounding the band 28 of flexible material. The band 28 of material has a first diameter that is substantially the same as the diameter of the first perimeter 26, and a second diameter substantially the same as the second perimeter 30. The diameter of the band of material decreases in a direction away from the surface 20 of the graft 14 from the second perimeter to the first perimeter. In some embodiments, the band 28 of flexible material may include a support frame 48 having a plurality of support units disposed about a surface of the band 28. The fenestration 12 may be disposed at the apex of the geometric shape. In some embodiments, the band of flexible material is configured to independently move between an interior surface of the graft and an exterior surface of the graft.

In some embodiments, the prosthesis 10 is intended for placement in the ascending thoracic aorta and to accommodate vessels that branch from the aorta, for example, the renal arteries, and into which a branch vessel prosthesis may be placed. However, the prosthesis 10 is not limited for use in the ascending thoracic aorta but may be used in other vessels of the body from which other vessels branch, such as the abdominal aorta, the descending thoracic aorta, as well as other body vessels.

Figure 1 shows an embodiment of a prosthesis 10 that is a stent graft. The prosthesis 10 includes graft material 14 having a generally tubular body comprising a proximal end 22, a distal end 24, and a lumen 18 extending through the prosthesis 10 to permit passage of blood flow from the proximal end 22 to the distal end 24. As shown in this embodiment, the proximal end of the prosthesis 10 has a generally undulating configuration. As will be discussed later in the application, this generally undulating configuration of the proximal end of the stent graft is designed to conform with the aortic root. In some embodiments, the prosthesis 10 may be tapered in order to accommodate narrowing at the sinotubular junction and distal portions of the ascending aorta.

The prosthesis 10 further comprises at least one stent coupled to the graft 14 that has a contracted delivery state and further has an expanded state for maintaining patency within a portion of the graft. The stents 16 may be placed on the external surface 20 and/or internal surface 21 of the graft material 14. In one particular embodiment, the prosthesis 10, such as that shown in Figure 1, has at least two external body stents 16a and 16b. Additionally, a sealing stent 45 may be placed at either or both the proximal and distal ends 22, 24 of the prosthesis 10. The stents 16 and 45 of the prosthesis 10 may be either self-expanding or balloon expandable. Preferably, they are self-expanding. However, a combination of self-expanding and balloon expandable stents 16 also may be contemplated. The prosthesis 10 may also include an aortic sinus stent assembly 50. The aortic sinus stent assembly 50 is configured to provide sufficient space for the native valve commissures and are curved and flared to match sinus shape to achieve conformance without disturbing native valve leaflet coaptation and hemodynamics.

In this embodiment, the sealing stent 45 is proximally positioned on the prosthesis 10. As shown, the sealing stent 45 is configured to be positioned at the sinotubular junction of a patient's aortic root. The sealing stent 45 may be flared to seal and prevent migration. Additionally, or alternatively, the sealing stent 45 may include fixation barbs to prevent antegrade or retrograde migration of the prosthesis. The sealing stent 45 is designed to mate with the aortic sinus stent assembly 50 in order to form a support frame for the fenestrations 12a and 12b. The stents 16 and 45 and the aortic sinus stent assembly 50 maybe covered or uncovered. In some embodiments, the stents 16 and 45 and the aortic sinus stent assembly 50 may be covered with methods such sewing a fabric graft material to the internal or external surface of the stents, or dipping or electrospinning a biocompatible material.

Stents 16 and 45 may be configured in the form of one or more "Z-stents", each of which may comprise a series of substantially straight segments 32 interconnected by a series of bent segments 36. The bent segments may comprise acute bends or apices. The stents are arranged in a zigzag configuration in which the straight segments 32, 34 are set at angles relative to each other and are connected by the bent segments. However, the stents 16 may comprise any suitable configuration and one or more stents 16 may be provided.

Stent amplitude, spacing and stagger are preferably optimized for each prosthesis design. In some embodiments, the apices or bends 36 of the struts 32, 34 may be staggered for minimal contact with each other. As shown in Figure 1, the stents 16a, 16b, and 45 are positioned adjacent each other and the apices 36 of each row are in circumferential alignment or "in phase", with the apices of longitudinally adjacent rows. In other embodiments, one or more of the stents 16 may be positioned "out of phase" by about 180 degrees with a longitudinally adjacent row, such that circumferentially about the surface of the graft, every other apex of the stents matches with every other apex of stent row 16d. In other embodiments, the stents 16 may be positioned in phase with a longitudinally adjacent row, or the stents may be out of phase by an amount less than 180 degrees.

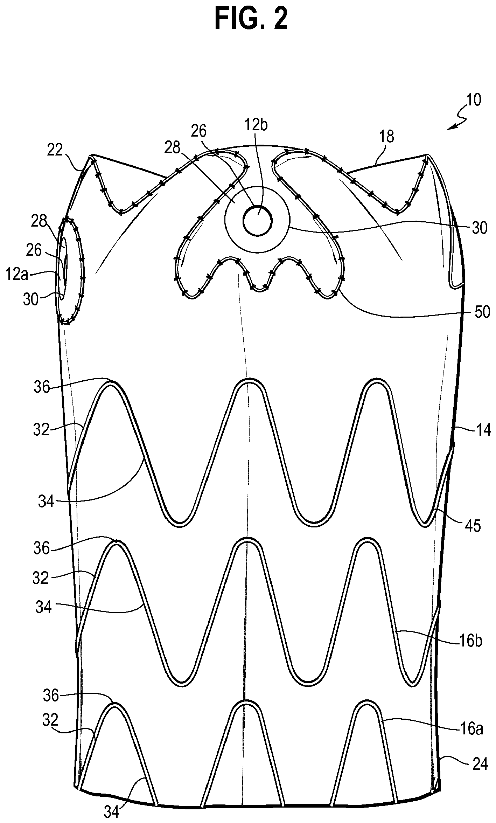

The prosthesis 10 has several openings or fenestrations that extend from the internal surface 21 to the external surface 20 of the graft material 14. The pivotable fenestrations 12 are positioned to align with, for example, the coronary arteries. In other embodiments, the one or more pivotable fenestrations 12 may be positioned to align with other branch arteries throughout a diseased vasculature. Additional fenestrations and scallops as disclosed here may also be included. As shown in Figures 1 and 2, the prosthesis 10 has two pivotable fenestrations. The pivotable fenestrations 12 have an inner perimeter 26 surrounding the fenestration 12, a band 28 surrounding the inner perimeter 26, and an outer perimeter 30 surrounding the band 28. As shown, the outer perimeter 30 diameter is greater than the band 28 diameter and the inner perimeter diameter 26. The inner perimeter 26, the band 28 and the outer perimeter 30 would be substantially concentric with one another if they were in the same plane, for example the surface plane of the graft. The pivotable fenestrations 12 may be located within the lumen 18 of the prosthesis 10 or extending from the exterior of the prosthesis 10. In the first example, the pivotable fenestrations 12 may be said to be concave, relative to the external surface 20 of the graft material 14. In the second example, the pivotable fenestrations 12 may be said to be convex, relative to the external surface 20 of the graft material 14. Figure 1 shows the pivotable fenestrations 12 located internal to the prosthesis 10, that is, they lie within the lumen 18 of the prosthesis 10.

The inner perimeter 26, the band 28 and the outer perimeter 30 may form a geometric shape, resembling, for instance, a frustoconical cone extending from the surface of the graft material 14. The fenestration 12 is provided at the peak or top of the geometric shape. In other embodiments, the band 28 may comprise a tapered, flexible tube extending from the outer perimeter 30 and the inner diameter 26. In this embodiment, the pivotable fenestrations 12 have a generally circular configuration. In other embodiments, the pivotable fenestrations 12 may have other suitable configurations, including, but not limited to, oblong, oval, rectangular, or triangular. In some embodiments, a support frame having a plurality of support units may surround the fenestration 12 and is positioned on a surface of the band 28. In the embodiment shown in Figures 1 and 2, a frame is not provided on the surface of the band. In some embodiments of the prosthesis 10 may include one pivoting fenestration and one non-pivoting fenestrations or two non-pivoting fenestrations. Other embodiments of the prosthesis 10 may include different configurations of fenestrations including, but not limited to, donut, pleated, diamond, or non-pivoting fenestrations.

As shown throughout the Figures, inner perimeter 26, band 28, and outer perimeter 30 surround the pivotable fenestration 12 to create a hemisphere shaped or frustoconical extension or protrusion. The outer perimeter 30 may be affixed to the graft material 14 by any attachment method including suturing circumferentially about an aperture disposed through graft material 14. The band 28 may be comprised of the same or different biocompatible material as the graft material 14. For example, the second biocompatible material may have greater pliability than the first biocompatible graft material used for the tubular graft body.

The band 28 is sufficiently flexible to permit the fenestration 12 to move such that a branch stent disposed in the fenestration 12 may be oriented upwardly, downwardly, laterally, diagonally and the like. In some embodiments, the band has up to about 180 degrees of freedom of movement relative to the surface plane of the prosthesis 10. Accordingly, the pivotable fenestration 12 allows the prosthesis 10 to be used with a variety of patients, due to its ability to adapt to the variance in the positioning of the diseased branch vessels. For example, if a body branch vessel is or becomes offset longitudinally or axially from a pivoting fenestration 12, the pivoting fenestration 12 will pivot the branch vessel prosthesis in the necessary direction and to the necessary degree to maintain the branch vessel prosthesis in place in the branch vessel. In some embodiments, the band 28 of flexible material is configured to independently move between an interior surface of the graft and an exterior surface of the graft.

Reinforcement members may be attached to the graft 14 surrounding the outer perimeter of the pivotable fenestrations 12. In one preferred embodiment, the reinforcement members comprise a wire that is sutured about the fenestrations 12a and 12b to reinforce the fenestration. The reinforcement members may be made of any suitable material. One preferred material is a superelastic or shape memory material, such as Nitinol. In another preferred embodiment, the reinforcement members may be made of radiopaque or other imageable material. In another embodiment the reinforcement members may be a wire that is looped about itself into a ring with unattached ends such that the ring may be expanded or contracted in diameter, such as described in co-pending U.S. Patent No. 8,808,351, herein incorporated by reference.

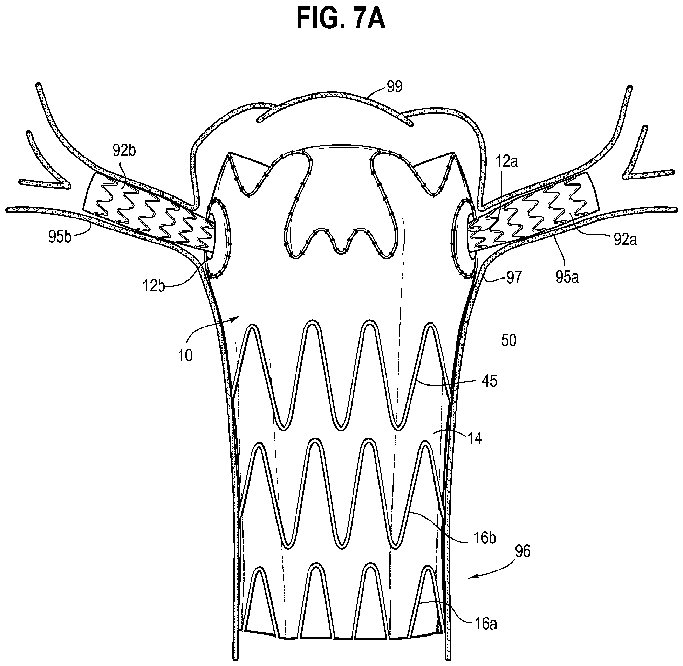

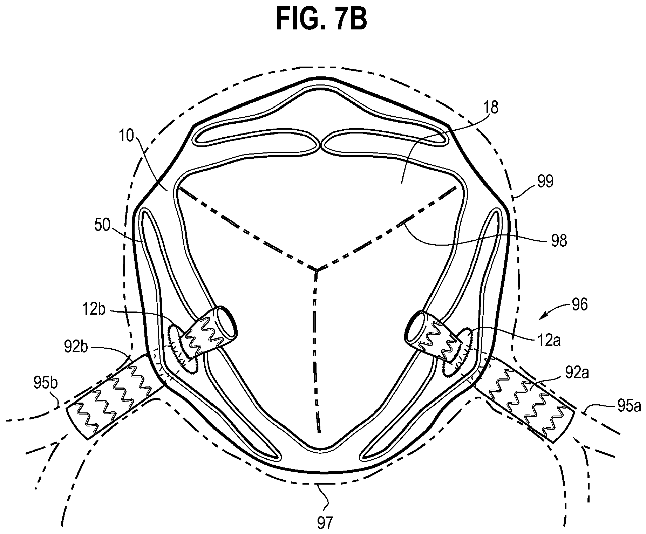

As shown in the Figures, the pivotable fenestrations extend or protrude into the lumen 18 of the prosthesis 10. As shown in Figure 2, the outer perimeter 30 lies substantially flush (in the same plane) of the graft material 14, and the band 28 and the outer perimeter 30 form a hemispherical shape, such as a dome, extending into the lumen 18. The first and second fenestrations may be disposed in graft 14 at locations between about 90 and about 270 degrees apart, though the positioning may be greater or less. In the deployed state, a first branch vessel prosthesis 92a extends between the first fenestration 12a and a first coronary artery 95a in a deployed state, and a second branch vessel prosthesis 94 extends between the second fenestration 12b and a second coronary artery 95b, as depicted in FIGS. 7A and 7B. Advantageously, if the first and second fenestrations 12a and 12b are not exactly aligned with the coronary arteries for any reason, such as variable patient anatomy, then the pivotable fenestrations 12a and 12b provide the requisite flexibility and ability to pivot so that the branch vessel prostheses 92 and 94 to deploy into the desired position.

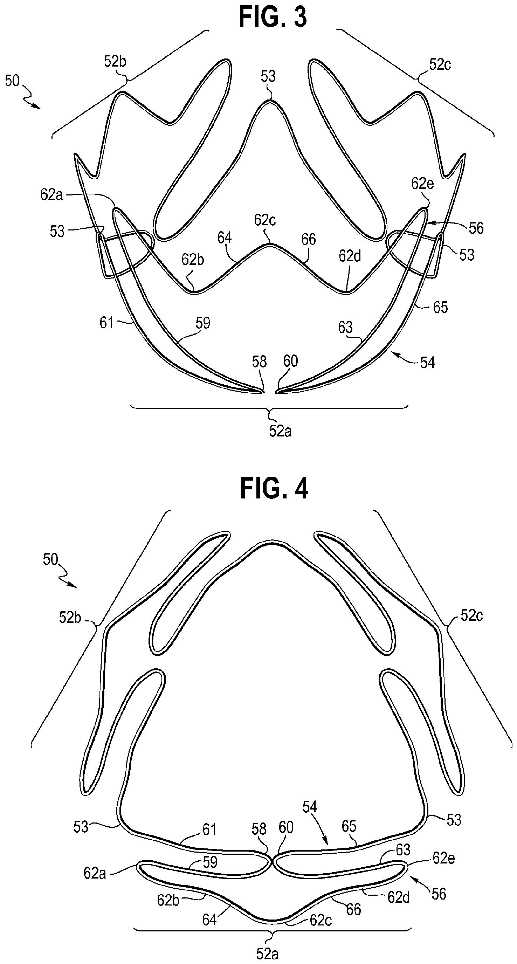

Figures 3 and 4 show an embodiment of aortic sinus stent assembly 50. In one embodiment, the aortic sinus stent assembly 50 is a continuous wire formed into one or more stent units 52a, 52b, 52c, where each stent unit 52a, 52b, 52c includes a proximal section 54 and a distal section 56. As shown, the proximal section 54 and the distal section 56 of the aortic sinus stent assembly 50 is non-symmetrical. In the present example, the aortic sinus stent assembly 50 includes three stent units. In other embodiments, the aortic sinus stent assembly 50 may include fewer than three stent units or more than three stent units. The proximal section 54 includes a first bend 58 and a second bend 60. As shown in the embodiment, a first curved strut 59 and a second curved strut 61 interconnect with first bend 58 of the aortic sinus stent assembly 50. Similarly, a third curved strut 63 and a fourth curved strut 65 interconnect with the second bend 60. The first curved strut 59 and the second curved strut 61, along with third curved strut 63 and the fourth curved strut 65, are curved such that the first bend 58 and the second bend 60 are facing one another and are circumferentially spaced apart from each other. In this embodiment, the first curved strut 59 and the second curved strut 61 have different lengths, where the first curved strut 59 is longer than the second curved strut 61. Likewise, the third curved strut 63 and the fourth curved struts 65 have different lengths, where the third curved strut 63 is longer than the fourth curved strut 65. The length of the struts may be modified in other embodiments to accommodate the size of a specific patient's aortic root. The first bend 58 and the second bend 60 have generally the same radii of curvature. The stent units 52a, 52b, 52c of the aortic sinus stent assembly 50 are interconnected by intermediate bends 53 positioned between each stent unit 52. As shown in Figure 4, the intermediate bend 53 interconnects the second curved strut 59 of one stent unit with the fourth curved strut 63 of a circumferentially adjacent stent unit 52. While the aortic sinus stent assembly is shown as a continuous wire, other embodiments of the aortic sinus stent assembly 50 may include separate stent units 52 that are joined together by techniques including, but not limited to, welding, adhesives, binding, and other techniques known to one of skill in the art.

The distal section 56 of the aortic sinus stent assembly 50 has a generally undulating shape and comprises a series of substantially straight segments 66 interconnected by a series of bent segments 62. The bent segments may comprise acute bends or apices. The distal section 56 of the aortic sinus stent assembly 50 is arranged in a zigzag configuration in which the straight segments 64, 66 are set at angles relative to each other and are connected by the bent segments 62. The distal section may have at least two apices or bends. In the present example shown in Figures 3 and 4, the distal section 56 comprises five bent segments 62 in the form of five apices. In other embodiments, the distal section 56 of the aortic sinus stent assembly may have less than or greater than five bent segments 62. The distal section 56 of the aortic sinus stent assembly 50 is connected to the proximal section 54 of the aortic sinus stent assembly 50 by the second curved strut 61 one side and the fourth curved strut 65 on the opposite side. The second curved strut 61 of the proximal section is interconnected to the first distal bent segment 62a and the fourth curved strut 65 is interconnected to the fifth distal bent segment 62e. As shown, the second distal bent segment 62b, the third distal bent segment 62c, and the fourth distal bent segment 62d have generally the same radii of curvature. Likewise, the first distal bent segment 62a and the fifth distal bent segment 62e have generally the same radii of curvature. In the present embodiment, the radii of curvature of the first distal bent segment 62a and the fifth distal bent segment 62e is different than the radii of curvature of the second distal bent segment 62bc, the third distal bent segment 62c, and the fourth distal bent segment 62d. In other embodiments, the second distal bent segment 62b, the third distal bent segment 62c, and the fourth distal bent segment 62d may have the same radii of curvature as the first distal bent segment 62a and the fifth distal bent segment 62e.

The prosthesis 10 is useful for treating type-A thoracic aortic dissection (TAD-A) with an entry tear at or within close proximity of the sinotubular junction. As discussed above, the aortic sinus stent assembly 50, when used with a prosthesis 10, is designed to mates with the sealing stent 45 to form in order to form a support frame for the fenestrations 12a and 12b. In addition, the aortic sinus stent assembly 50 advantageously does not affect a patient's native aortic valve because it is configured and designed to fit behind the valve leaflets in the aortic sinus. The aortic sinus stent assembly 50 also assists in aligning the fenestrations with the coronary ostia as well as prevent retrograde migration, due to the non-symmetrical configuration. Furthermore, the curved struts of the proximal section 54 of the aortic sinus stent assembly 50 positions the first bend 58 and the second bend 60 near the midpoint of the distal section 56 of the aortic sinus stent assembly 50. This configuration allows the aortic sinus stent assembly 50 to be loaded into a smaller French size sheath and prevents permanent deformation during loading of the prosthesis 10 on a delivery device 10.

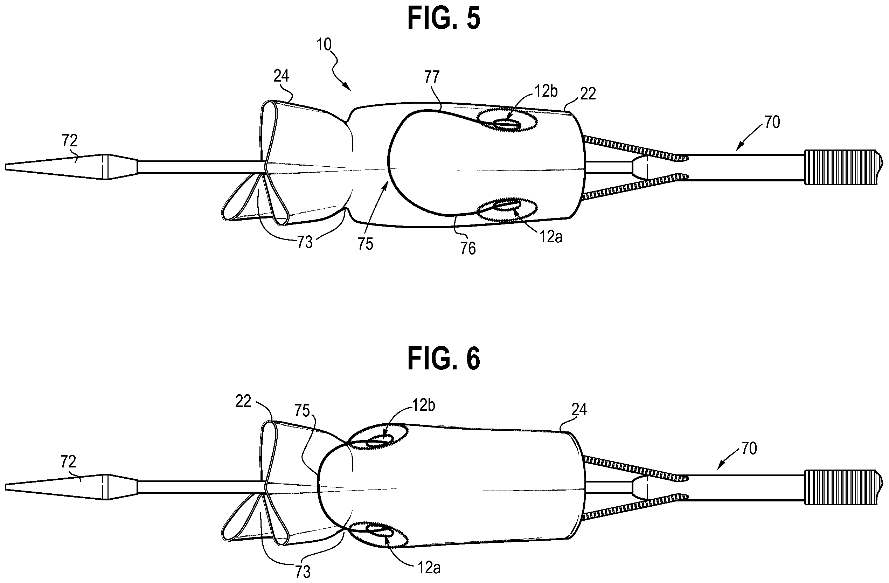

In the examples of FIGS. 1-8, the deployment of the prosthesis 10 into the state shown in FIG. 7A and 7B may be achieved in different manners. In one example, the deployment may be made using a transapical or transeptal approach, in which case the prosthesis 10 may be secured to an exemplary delivery system 70 as shown in FIG. 5. In the transapical or transeptal approach, an atraumatic tip 72 of the delivery system is advanced in an antegrade fashion, i.e., in a direction from the aortic annulus 97 towards the ascending aorta 98.

In another example, the deployment may be made using a femoral, carotid, subclavian or auxiliary approach, in which case the prosthesis 10 may be secured to the exemplary delivery system 20 as shown in FIG. 6. In this approach, the atraumatic tip 72 of the delivery system 70 is advanced in a retrograde fashion, i.e., in a direction from the ascending aorta 98 towards the aortic annulus 97. In either delivery approach, as shown in FIGS. 5 and 6, the graft 14 may comprise one or more regions 13 that are radially restrained.

Further, the prosthesis 10 may be provided as part of a preloaded system that includes a guide wire 75. In this example, a first end segment 76 of the guide wire 276 may enter the lumen 18 through a proximal or distal end of the prosthesis 10, depending on the delivery orientation of the prosthesis shown in FIG. 5 as compared to FIG. 6. The first end segment 76 exits the graft 14 through the first fenestration 12a. An intermediate segment of the guide wire 75 may extend external of the graft 14 and reenter the lumen 18 of the prosthesis 10 through the second fenestration 12b. A second end segment 77 of the guide wire 75 may extend distally within the lumen 218 and may extend distally to the distal end of the delivery device 270. The first end segment 76 of the guide wire 75 may enable introduction of the first branch prosthesis 92a into the first fenestration 12a to couple the prosthesis 10 to the right coronary artery, and the second end segment 77 of the guide wire 77 may enable introduction of the second branch prosthesis 92b into the second fenestration 12b to couple the prosthesis to the left coronary artery.

Figure 7A and 7B depict shows the prosthesis 10 positioned within the ascending aorta 96 and the aortic root 97. The prosthesis 10 has been placed in a location providing patent fluid flow away from and isolating a dissection. The aortic sinus stent assembly 50 engages the cusp of each aortic sinus 99 of the aortic root adjacent to the aortic valve 80 and proximal to the first coronary artery 95a and the second coronary artery 95b. A first branch prosthesis 92a is deployed through the first fenestration 12a to couple the prosthesis 10 to the right coronary artery, and a second branch prosthesis 92b is deployed through the second fenestration 12b to couple the prosthesis 10 to the left coronary artery 95b. As will be appreciated by those of skill in the art, the region being treated is subjected to movement, fluid pressure, and turbidity from blood flow. Anchoring of the aortic sinus stent assembly 50 in the natural concavity of the aortic root 97 will prevent migration of the stent graft in a manner that could impair blood flow to/through the coronary arteries 95a and 95b and/or the proper functioning of the aortic valve 98, as shown by Figure 7B. The aortic valve 98 is unimpaired and blood flow through the aortic root 97 and the lumen 18 of the prosthesis 10 is unobstructed. The sealing stent 45 is engaged with the sinotubular junction, which will prevent migration that could impair blood flow to/through the vessels. The intermediate stents 16a and 16b (one or more of which may be barbed) preferably anchor the graft material against the aorta wall in a manner maintaining surface contact to isolate the dissection.

As noted above, while one exemplary use of the prosthesis 10 has been shown with regard to the aortic sinus and ascending aorta, the prosthesis 10 may be deployed in other parts of a patient's arterial or venous system, or any suitable duct, passageway or vessel.

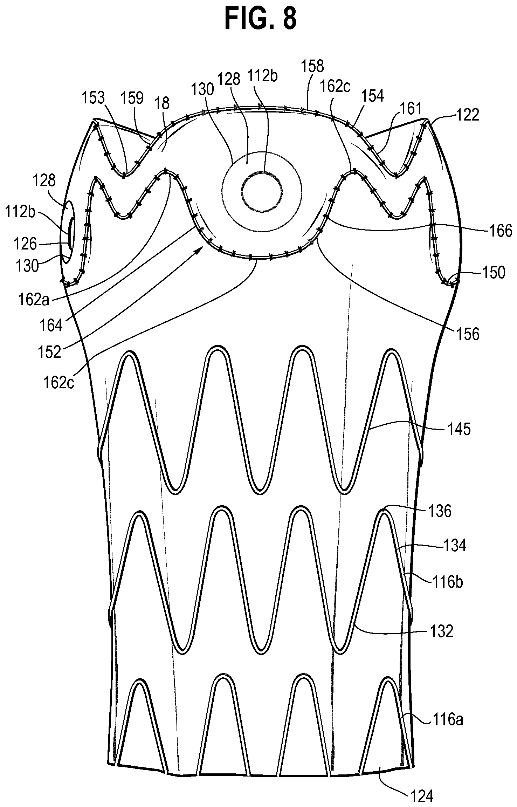

Figure 8 shows an embodiment of a prosthesis 110 that is a stent graft. The prosthesis 110 includes graft material 114 having a generally tubular body comprising a proximal end 122, a distal end 124, and a lumen 118 extending through the prosthesis 10 to permit passage of blood flow from the proximal end 122 to the distal end 124. As shown in this embodiment, the proximal end of the prosthesis 10 has a generally undulating configuration. This generally undulating configuration of the proximal end of the stent graft is designed to conform with the aortic root.

The prosthesis 110 further comprises at least one stent coupled to the graft 114 that has a contracted delivery state and further has an expanded state for maintaining patency within a portion of the graft. The stents 116 may be placed on the external surface 120 and/or internal surface 121 of the graft material 114. In one particular embodiment, the prosthesis 110 has at least two external body stents 116a and 116b. Additionally, a sealing stent 145 may be placed at either or both the proximal and distal ends 122, 124 of the prosthesis 110. The stents 116 and 145 of the prosthesis 110 may be either self-expanding or balloon expandable. Preferably, they are self-expanding. However, a combination of self-expanding and balloon expandable stents 116 also may be contemplated. The prosthesis 110 may also include an aortic sinus stent assembly 150. The aortic sinus stent assembly 50 is configured to provide sufficient space for the native valve commissures and are curved and flared to match sinus shape to achieve conformance without disturbing native valve leaflet coaptation and hemodynamics.

In this embodiment, the sealing stent 145 is proximally positioned on the prosthesis 10. As shown, the sealing stent 145 is configured to be positioned at the sinotubular junction of a patient's aortic root. The sealing stent 145 may be flared to seal and prevent migration. Additionally, or alternatively, the sealing stent 145 may include fixation barbs to prevent antegrade or retrograde migration of the prosthesis. The sealing stent 45 is designed to mate with the aortic sinus stent assembly 150 in order to form a support frame for the fenestrations 112a and 112b. The stents 116 and 145 and the aortic sinus stent assembly 150 maybe covered or uncovered. As discussed with previous embodiments, stents 116 and 145 may be configured in the form of one or more "Z-stents". However, the stents 116 may comprise any suitable configuration and one or more stents 116 may be provided.

As shown in Figure 8, the stents 16a, 16b, and 45 are positioned adjacent each other and the apices 36 of each row are in circumferential alignment or "in phase", with the apices of longitudinally adjacent rows. In other embodiments, one or more of the stents 16 may be positioned "out of phase" by about 180 degrees with a longitudinally adjacent row, such that circumferentially about the surface of the graft, every other apex of the stents matches with every other apex of stent row 16d. In other embodiments, the stents 16 may be positioned in phase with a longitudinally adjacent row, or the stents may be out of phase by an amount less than 180 degrees.

The prosthesis 110 has several openings or fenestrations that extend from the internal surface 21 to the external surface 20 of the graft material 14. The pivotable fenestrations 12a and 12b are positioned to align with, for example, the coronary arteries. The pivotable fenestrations 12a and 12b have an inner perimeter 26 surrounding the fenestration 112, a band 128 surrounding the inner perimeter 126, and an outer perimeter 130 surrounding the band 128. As shown, the outer perimeter 130 diameter is greater than the band 28 diameter and the inner perimeter diameter 126. The inner perimeter 126, the band 128 and the outer perimeter 130 would be substantially concentric with one another if they were in the same plane, for example the surface plane of the graft. The pivotable fenestrations 112 may be located within the lumen 118 of the prosthesis 110 or extending from the exterior of the prosthesis 110. In the first example, the pivotable fenestrations 112 may be said to be concave, relative to the external surface 120 of the graft material 114. The inner perimeter 126, the band 128 and the outer perimeter 130 may form a geometric shape, resembling, for instance, a frustoconical cone extending from the surface of the graft material 114. The fenestration 112 is provided at the peak or top of the geometric shape.

The aortic sinus stent assembly 150 is a continuous wire formed into one or more stent units 152 including a proximal section 154 and a distal section 156. As shown, the proximal section 154 and the distal section 156 of the aortic sinus stent assembly 150 is non-symmetrical. In the present example, the aortic sinus stent assembly 150 includes three stent units. In other embodiments, the aortic sinus stent assembly 150 may include fewer than three stent units or more than three stent units. The proximal section 154 includes a first bend 158 interconnecting a first strut 159 and second strut 161. The stent units 152a, 152b, 152c of the aortic sinus stent assembly 150 are interconnected by intermediate bends 153 positioned between each stent unit 152. The intermediate bend 53 interconnects the second strut 159 of one stent unit with the first strut of a circumferentially adjacent stent unit 152. While the aortic sinus stent assembly is shown as a continuous wire, other embodiments of the aortic sinus stent assembly 150 may include separate stent units 152 that are joined together by techniques including, but not limited to, welding, adhesives, binding, and other techniques known to one of skill in the art.

The distal section 156 of the aortic sinus stent assembly 150 has a generally undulating shape and comprises a series of substantially straight segments 66 interconnected by a series of bent segments 162. The bent segments may comprise acute bends or apices. The distal section 156 of the aortic sinus stent assembly 150 is arranged in a zigzag configuration in which the straight segments 164, 166 are set at angles relative to each other and are connected by the bent segments 162. As shown, the first distal bent segment 162b and the third distal bent segment 162c have generally the same radii of curvature. In the present embodiment, the radii of curvature of the second distal bent segment 162b is different than the radii of curvature of the first distal bent segment 162a and the third distal bent segment 162c. The first bend 158 of the proximal section 154 and the second distal bent segment of the distal section are configured to provide additional support and surface area for the one or more fenestrations 112 of the prostheses 110. In some embodiments, the stent units 152 may be formed by a continuous wire. Other embodiments of the aortic sinus stent assembly 150 may include separate stent units 152 that are joined together by techniques including, but not limited to, welding, adhesives, binding, and other techniques known to one of skill in the art

While various embodiments of the invention have been described, the invention is not to be restricted except in light of the attached claims and their equivalents. Moreover, the advantages described herein are not necessarily the only advantages of embodiments of the invention and it is not necessarily expected that every embodiment of the invention will achieve all of the advantages described.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application number 62/553,235, from which this application claims priority, and in the abstract accompanying this application are incorporated herein by reference.

## Claims

1. A prosthesis for placement within an aortic root, comprising:
a graft having a proximal end, distal end, and lumen disposed therethrough,
a stent assembly disposed at that proximal end of the graft, the stent assembly comprising a first stent unit comprising a first proximal section having a first bend interconnected by a first curved strut and a second curved strut and a second bend interconnected by a third curved strut and a fourth curved strut, the first bend of the first proximal section and second bend of the first proximal section facing each other and circumferentially spaced apart; and a first distal section connected to the first proximal section.

2. The prosthesis of claim 1, wherein the first distal section comprises a plurality of structural struts connected by apices; wherein optionally the first distal section comprises at least two apices; wherein optionally the first distal section comprises five apices.

3. The prosthesis of any preceding claim, further comprising a second stent unit comprising a second proximal section circumferentially adjacent to the first proximal section, the second proximal section comprising a first bend interconnected by a first curved strut and a second curved strut and second bend interconnected by a third curved strut and a fourth curved strut, the first bend of the second proximal section and the second bend of the second proximal section facing each other and circumferentially disposed.

4. The prosthesis of claim 3, wherein the second curved strut of the first proximal section and the fourth curved strut of the second proximal section are interconnected, optionally by a first intermediate bend.

5. The prosthesis of claim 3 or 4, further comprising a third stent unit comprising a third proximal section circumferentially adjacent to the first proximal section and the second proximal section, the third proximal section comprising a first bend and second bend, the first bend and the second bend facing each other and circumferentially disposed.

6. The prosthesis of claim 5, wherein the fourth curved strut of the first proximal section and the second curved strut of the third proximal section are interconnected, optionally by a second intermediate bend, and wherein the second curved strut of the second proximal section and the fourth curved strut of the third proximal section are interconnected, optionally by a third intermediate bend.

7. The prosthesis of any preceding claim, further comprising at least one fenestration disposed through a side wall of the graft; wherein the at least one fenestration is optionally positioned between the first proximal section and the first distal section of the stent assembly; wherein the at least one fenestration is optionally pivotable in any direction away from an axis perpendicular to a longitudinal axis of the prosthesis.

8. The prosthesis of any preceding claim, further comprising:
a first perimeter having a first diameter and surrounding the at least one fenestration;
a band of flexible material surrounding the first perimeter;
a second perimeter attached to and surrounding the band of flexible material and having a second diameter greater than the first diameter of the first perimeter; and
wherein the band of flexible material has a first diameter substantially the same as the first diameter of the first perimeter and a second diameter substantially the same as the second diameter of the second perimeter, and where the diameter of the band of flexible material decreases in a direction away from a surface of the graft from the second perimeter to the first perimeter;
wherein optionally the band of flexible material is configured to independently move between an interior surface of the graft and an exterior surface of the graft.

9. The prosthesis of any preceding claim, wherein
the first distal section comprises a plurality of structural struts connected by apices.

10. A stent for an aortic valve sinus, comprising,
a first stent unit comprising a first proximal section having a first bend interconnected by a first curved strut and a second curved strut and a second bend interconnected by a third curved strut and a fourth curved strut, the first bend of the first proximal section and second bend of the first proximal section facing each other and circumferentially spaced apart; and
a first distal section comprising a plurality of structural struts connected by apices.

11. The stent of claim 10, wherein the first distal section comprises at least two apices.

12. The stent of claim 10 or 11, further comprising a second stent unit comprising a second proximal section circumferentially adjacent to the first proximal section, the second proximal section comprising a first bend interconnected by a first curved strut and a second curved strut and second bend interconnected by a third curved strut and a fourth curved strut, the first bend of the second proximal section and the second bend of the second proximal section facing each other and circumferentially disposed;
wherein optionally the second curved strut of the first proximal section and the fourth curved strut of the second proximal section are interconnected.

13. The stent of claim 12, further comprising a third stent unit comprising a third proximal section circumferentially adjacent to the first proximal section and the second proximal section, the third proximal section comprising a first bend and second bend, the first bend and the second bend facing each other and circumferentially disposed;
wherein optionally the fourth curved strut of the first proximal section and the second curved strut of the third proximal section are interconnected and wherein optionally the second curved strut of the second proximal section and the fourth curved strut of the third proximal section are interconnected.

14. The prosthesis of any preceding claim, comprising:
a valve replacement positioned between the proximal end of the graft and the distal end of the graft;
wherein the stent assembly comprises at least one proximal section comprising a first proximal apex interconnected by struts and a second proximal apex interconnected by struts.

15. A prosthesis for placement within an aortic valve, comprising:
a graft having a proximal end, a distal end, and a lumen disposed therethrough;
a valve replacement positioned between the proximal end of the graft and the distal end of the graft; and
a stent assembly disposed at the proximal end of the graft, the stent assembly comprising at least one proximal section, the proximal section comprising a first proximal apex interconnected by struts and a second proximal apex interconnected by struts.
